# EUROPEAN PATENT APPLICATION

(11) **EP 3 811 901 A1**
(43) Date of publication of application: **28.04.2021**
(21) Application number: 19821842.2
(22) Date of filing: 06.06.2019
(51) Int. Cl.: A61F 2/06, B29C 64/20

(54) **MANUFACTURING METHOD FOR LUMINAL TISSUE CONSTRUCT**

(30) Priority: 19.06.2018 CN 201810629319
(71) Applicant: Revotek Co., Ltd, Sichuan 611731 (CN)
(72) Inventor: LI, Yijun, Chengdu, Sichuan 611731 (CN); HE, Junxuan, Chengdu, Sichuan 611731 (CN); HU, Xiaolin, Chengdu, Sichuan 611731 (CN); JIANG, Zhi, Chengdu, Sichuan 611731 (CN); ZUO, Xiao, Chengdu, Sichuan 611731 (CN)
(74) Representative: Kolster Oy Ab
(86) International application number: PCT/CN2019/090374
(87) International publication number: WO 2019/242509

(57) **Abstract**

The present disclosure relates to a manufacturing method of a lumen tissue construct. The manufacturing method of a lumen tissue construct comprises: step 1: installing elastic film on a support in a sleeving manner; step 3: printing a biological construct on the elastic film; step 5: forming a construct bonding layer on the biological construct; and step 7: bonding a lumen tissue to the construct bonding layer. In the manufacturing method of the lumen tissue construct of the present disclosure, the printed biological construct is assembled on the inner surface of the lumen tissue to form the lumen tissue construct, thereby helping to avoid occurrence of problems such as recurrence of thrombus, restenosis of lumen and the like after the lumen tissue construct is implanted for a long time, and improving the biological reliability of the lumen tissue construct.

## Description

### RELATED APPLICATION

The present application is based on and claims priority to Chinese patent application No. 201810629319.9 entitled "Manufacturing Method of Lumen Tissue Construct" filed on June 19, 2018, the disclosure of which is hereby incorporated in its entirety.

### TECHNICAL FIELD

The present disclosure relates to the technical field of 3D bioprinting, and particularly relates to a manufacturing method of a lumen tissue construct.

### BACKGROUND

In the prior art, common artificial blood vessels are made from polymer fibers (such as nylon, and polyester), silk or expanded polytetrafluoroethylene. When a blood vessel is transplanted, a complete artificial blood vessel can be used to replace a diseased or damaged blood vessel. Although the use of such artificial blood vessels to replace diseased or damaged blood vessels has achieved great results clinically, it still faces problems difficult to solve, including recurrence of thrombus and appearance of lumen restenosis after the blood vessels are implanted for a long time. The root cause of these problems is the lack of a complete layer of endothelial cells on the inner surface of this artificial blood vessel.

In addition, since the artificial blood vessel can hardly be deformed in the radial direction, the prior art cannot compress the artificial blood vessel externally to make bio-blocks be totally evenly, completely, and smoothly attached to the inner wall of the artificial blood vessel.

### SUMMARY OF THE INVENTION

The present disclosure provides a manufacturing method of a lumen tissue construct, comprising:
step 1: installing elastic film on a support in a sleeving manner;
step 3: printing a biological construct on the elastic film;
step 5: forming a construct bonding layer on the biological construct; and
step 7: bonding a lumen tissue to the construct bonding layer.

In some embodiments, the step 3 comprises:
step 31: providing at least one bioprinting unit;
step 33: forming a printing unit bonding layer on a predetermined area of the elastic film; and
step 35: bonding the bioprinting unit to the printing unit bonding layer to form a biological construct or an intermediate structure of the biological construct.

In some embodiments, forming the printing unit bonding layer on the predetermined area of the elastic film comprises: forming an isolation medium layer in the predetermined area of the elastic film, and then the printing unit bonding layer is formed on the elastic film by virtue of the isolation medium layer.

In some embodiments, the step 3 further comprises:
step 37: forming a printing unit bonding layer on the intermediate structure obtained in the previous step to obtain an intermediate structure having the printing unit bonding layer; and
step 39: bonding the bioprinting unit to the intermediate structure having the printing unit bonding layer.

In some embodiments, the step 3 further comprises repeating the step 37 and the step 39 one or more times.

In some embodiments, the step 5 comprises: forming a supporting layer on the surface of the biological construct; and then forming a construct bonding layer on the biological construct by virtue of the support layer.

In some embodiments, the step 5 comprises: printing a bonding agent on the biological construct to form the construct bonding layer.

In some embodiments, the biological construct is a lumen structure, and the support is a rotating rod.

In some embodiments, the step 7 comprises:
installing the lumen tissue outside a hollow rod in a sleeving way;
installing the hollow rod outside the biological construct provided with the construct bonding layer in a sleeving way;
retaining the lumen tissue outside the biological construct while pulling out the hollow rod; and
bonding the lumen tissue to the construct bonding layer.

In some embodiments, bonding the lumen tissue to the construct bonding layer comprises: expanding the elastic film to increase the outer diameter of the biological construct and the outer diameter of the construct bonding layer so that the construct bonding layer is attached to the inner wall of the lumen tissue.

In some embodiments, gas is delivered from the inside of the support to the surface of the support to expand the elastic film.

In some embodiments, printing the biological construct on the elastic film further comprises heating the support.

In some embodiments, the method further comprises detecting the flatness of the inner wall of the biological construct.

Therefore, based on the above-mentioned technical scheme, in the manufacturing method of the lumen tissue construct of the present disclosure, the printed biological construct is assembled on the inner surface of the lumen tissue to form the lumen tissue construct, thereby helping to avoid occurrence of problems such as recurrence of thrombus, restenosis of lumen and the like after the lumen tissue construct is implanted for a long time, and improving the biological reliability of the lumen tissue construct.

### BRIEF DESCRIPTION OF DRAWINGS

The drawings described herein are used to provide a further understanding of the present disclosure and form a part of the present application. The exemplary embodiments and descriptions of the present disclosure are only used to explain the present disclosure and do not constitute an undue limitation on the present disclosure. In the drawings:
Fig.1 is a schematic diagram of the overall structure of a lumen tissue construct printing device embodiment that is adopted in the manufacturing method of the lumen tissue construct of the embodiment of the present disclosure;
Fig.2 is a schematic diagram of the overall structure of a nozzle assembly in the lumen tissue construct printing device of the embodiment of the present disclosure;
Fig.3 is a schematic diagram of the internal structure of the nozzle assembly in the lumen tissue construct printing device of the embodiment of the present disclosure;
Fig.4 is a schematic diagram of the cross-sectional structure of a screw pump embodiment in the lumen tissue construct printing device of the embodiment of the present disclosure;
Fig.5 is a schematic diagram of the cross-sectional structure of a bio-block nozzle embodiment in the lumen tissue construct printing device of the embodiment of the present disclosure;
Fig.6 is a partially enlarged schematic diagram of the circled part in Fig.5;
Fig.7 is a schematic diagram of the overall structure of a bioprinting platform in the lumen tissue construct printing device of the embodiment of the present disclosure;
Fig.8 is a schematic diagram of the local structure of the bioprinting platform in the lumen tissue construct printing device of the embodiment of the present disclosure;
Fig.9 is a schematic diagram of the structure of the rotating rod in the lumen tissue construct printing device of the embodiment of the present disclosure;
Fig.10 is a schematic diagram of the structure of a heating part in the lumen tissue construct printing device of the embodiment of the present disclosure;
Fig.11 is a schematic diagram of the structure in which the heating part is disposed inside the rotating rod in the lumen tissue construct printing device of the embodiment of the present disclosure;
Fig.12 is a schematic diagram of the internal structure of a rotating part in the lumen tissue construct printing device of the embodiment of the present disclosure;
Fig.13 is a schematic diagram of the structure of a first embodiment of a clamping mechanism in the lumen tissue construct printing device of the embodiment of the present disclosure;
Fig.14 is a schematic diagram, in another perspective, of the structure of a first embodiment of the clamping mechanism in the lumen tissue construct printing device of the embodiment of the present disclosure;
Fig.15 is a schematic diagram of the structure of a second embodiment of the clamping mechanism in the lumen tissue construct printing device of the embodiment of the present disclosure;
Fig.16 is a schematic diagram of the structure in which the lumen tissue is clamped by the second embodiment of the clamping mechanism in the lumen tissue construct printing device of the embodiment of the present disclosure;
Fig.17 is a schematic diagram of the structures of the construct bonding layer and the elastic film that sleeves the support in forming the isolation medium layer step of the biological construct printing step of a lumen tissue construct printing method of the embodiment of the present disclosure;
Fig.18 is a schematic diagram of a printing process of a first printing method in the biological construct printing step in the embodiment of the present disclosure;
Fig.19 is a schematic diagram of a printing process of a second printing method in the biological construct printing step in the embodiment of the present disclosure;
Fig.20 is a schematic diagram of a process that the bioprinting unit is grabbed and placed by a sucking component in the biological construct printing step in the embodiment of the present disclosure;
Fig.21 is a schematic diagram of the structure of the biological construct printing device that is adopted in the biological construct printing step in the embodiment of the present disclosure;
Fig.22 is a schematic diagram of the structure of a driving positioning component in the biological construct printing device embodiment of the present disclosure;
Fig.23 is a schematic diagram of the structure of a first nozzle assembly in the biological construct printing device embodiment of the present disclosure;
Fig.24 is a schematic diagram of the structure of a second nozzle assembly in the biological construct printing device embodiment of the present disclosure;
Fig.25 is a schematic diagram of the structure of a printing unit kit in the biological construct printing device embodiment of the present disclosure;
Fig.26 is a schematic diagram of the structure, from an overlook perspective, of the printing unit kit in the biological construct printing device embodiment of the present disclosure;
Fig.27 is a schematic diagram of the structure of a rotary biochemical analyzer in the biological construct printing device embodiment of the present disclosure; and
Fig.28 is a schematic diagram of the process of the manufacturing method of the lumen tissue construct of the embodiment of the present disclosure.

### DEDAILED DESCRIPTION

The technical schemes of the present disclosure will be further described in detail through the drawings and embodiments below.

The specific implementation modes of the present disclosure are intended to facilitate further explanation of the concept of the present disclosure, the technical problems solved, the technical features constituting the technical schemes, and the technical effects brought. It should be noted that the description of these implementation modes does not constitute a limitation of the present disclosure. In addition, the technical features involved in the implementation modes of the present disclosure described below can be combined with each other as long as they have no conflict with each other.

In the present disclosure, the term "biological construct" refers to a two-dimensional or three-dimensional structure artificially constructed and containing cells. In certain preferred implementation schemes, the biological construct is a three-dimensional construct, a tissue precursor, an artificial tissue, or an artificial organ.

The term "bioprinting unit" refers to a basic unit for constructing a biological construct by the methods and devices of the present disclosure, which can be used in multiple fields, for example, fields of bioprinting (such as 3D bioprinting), tissue engineering, regenerative medicine, and the like.

In some embodiments, the bioprinting unit is a microcapsule.

In some embodiments, the microcapsules are bio-blocks.

In some embodiments, the bio-block of the present disclosure may have a structure and composition as follows: a nucleus layer containing cells,
wherein the cells can grow, proliferate, differentiate or migrate, the nucleus layer is made from a biodegradable material and provides necessary materials for the life activities of the cells; and, a shell layer encapsulating the nucleus layer, wherein the shell layer is located outside, is made of a biodegradable material and provides mechanical protection for the inner nucleus layer and cells, and the bio-blocks of this structure can be used as a basic unit for biological 3D printing.

In some embodiments, the particle size of the bioprinting unit of the present disclosure is 0.5 mm to 3 mm.

In some embodiments, the bioprinting unit of the present disclosure is gel-state particles; the nucleus layer and/or shell layer of the bioprinting unit (bio-block) prepared by the method and the device of the present disclosure may be in a gel state. The bioprinting unit of the present disclosure may contain hydrogel.

In some embodiments, the hydrogel comprises alginate, agarose, gelatin, chitosan, or other water-soluble or hydrophilic polymers.

In some embodiments, the bioprinting unit has a stable structure under a physiological environment (for example, at a temperature between 4 °C to 37 °C, for example, at a pH between 6-8), and it is bio-blocks in some embodiments. The bioprinting unit may be plural and exists in the form of a mixture, which may each independently be spherical, or be in any desired shape (for example, a cube, a rectangular prism, a hexagonal prism, a cylinder, or an irregular shape). In some embodiments, the dimensions of the bioprinting units are each independently 20-2000 µm, for example, 30-1900 µm, 40-1800 µm, 50-1700 µm, 60-1600 µm, 70-1500 µm, 80-1400 µm, 90-1300 µm, 100- 1200µm, 200-1000µm, 300-800µm, 400-600µm, and 100-500µm. The bioprinting units may each independently be solid or semisolid, for example, in a gel state.

The bioprinting unit may exist in the form of a mixture. The bioprinting unit may be a separate microcapsule. The bioprinting unit may be disposed in a container.

As used herein, the term "microcapsule" refers to a microstructure (for example, a micron-to-millimeter-scale structure) containing cells and biocompatible materials, wherein the cells are encapsulated inside biocompatible materials. The microcapsules of the present disclosure have a stable structure under a physiological environment (for example, at a temperature between 4 °C to 37 °C, for example, at a pH between 6-8, for example, under fluid shear force in the physiological environment). In some embodiments, the microcapsules have mechanical strength that will not cause breakage of the microcapsules during suction or squeezing.

As used herein, the term "tissue" refers to cell aggregates included a group of cells that are the same or similar in shape and the same in function, and usually contains non-cellular-morphological substances (referred to as intercellular substance, such as matrix, fibers, etc.). The tissue may comprise one or more types of cells.

As used herein, the term "artificial tissue" refers to a tissue that is not formed by natural tissue generation or development processes. The artificial tissue may be an artificially produced tissue, for example, a tissue obtained by cultivating an artificial tissue precursor.

As used herein, the term "artificial tissue precursor" refers to an object that includes a support and a plurality of microcapsules of the present disclosure, wherein at least one microcapsule is bonded to the support. In certain implementation schemes, the artificial tissue precursor includes a support and a biological construct constructed from microcapsules. In certain implementation schemes, the artificial tissue precursor of the present disclosure can form an artificial tissue after operation steps such as culturing, induction and the like.

As used herein, the term "bonding" means that no relative displacement occurs. In certain implementation schemes, the microcapsules or the biological construct is bonded to the support, which means that the microcapsule or the biological construct is combined with the support.

As used herein, the term "support" refers to an object having a certain shape and being bonded to the microcapsules or a biological construct included the microcapsules in the artificial tissue precursor of the present disclosure. The support can provide a corresponding area for the biological construct to be completely fixed (adhered) to it.

As used herein, the term "lumen" refers to an organ that is tubular in shape and has a hollow inner chamber, for example, circulatory lumen, digestive lumen, respiratory lumen, urinary lumen or reproductive lumen, for example, blood vessels, esophagus, trachea, stomach, bile duct, and intestine (including small intestine and large intestine, for example, duodenum, jejunum, ileum, cecum (including appendix), ascending colon, right colon, transverse colon, left colon, descending colon, sigmoid colon, and rectum), oviducts, sperm ducts, ureters, bladder, or lymphatics.

As used herein, the term "artificial blood vessel" refers to an artificially produced blood vessel replacement, which is generally tubular. In certain implementation schemes, the artificial blood vessel is used to reconstruct or repair a narrowed, occluded, dilated, injured, or deformed blood vessel. In certain implementation schemes, the artificial blood vessel is obtained by culturing the tubular artificial tissue precursor of the present disclosure.

As used herein, the term "biocompatible material" refers to: a material (and its degradation products) that are non-toxic to cells, and are compatible with the host after being implanted into the host (such as a human body), will not cause significant or serious side effects, for example, will not cause toxic effects on the host (such as human tissues), and will not cause immune rejection, allergic or inflammatory reactions, etc. of the host.

As used herein, the term "biodegradable material" refers to a material that can be degraded and absorbed by cells or organisms, and its degradation products are biocompatible. Such material may be from natural sources (for example, from animals and plants), or may be artificially synthesized.

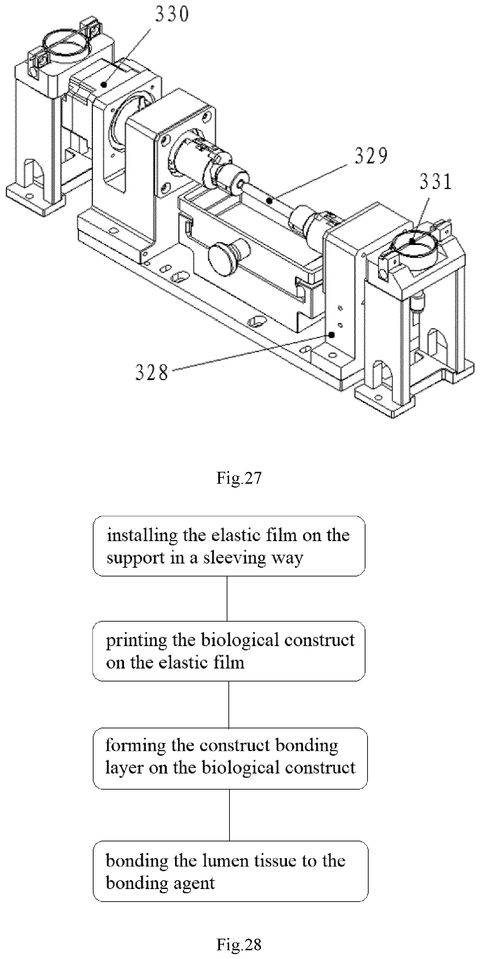

As shown in Fig.28, the present disclosure provides a manufacturing method of a lumen tissue construct, including:
step 1: installing elastic film on a support in a sleeving way;
step 3: printing a biological construct on the elastic film;
step 5: forming a construct bonding layer on the biological construct; and
step 7: bonding the lumen tissue to the construct bonding layer.

The manufacturing method of the lumen tissue construct of the present disclosure assembles the printed biological construct on the inner surface of the lumen tissue to form the lumen tissue construct, thereby helping to avoid occurrence of problems such as recurrence of thrombus, restenosis of lumen and the like after the lumen tissue construct is implanted for a long time, and improving the biological reliability of the lumen tissue construct.

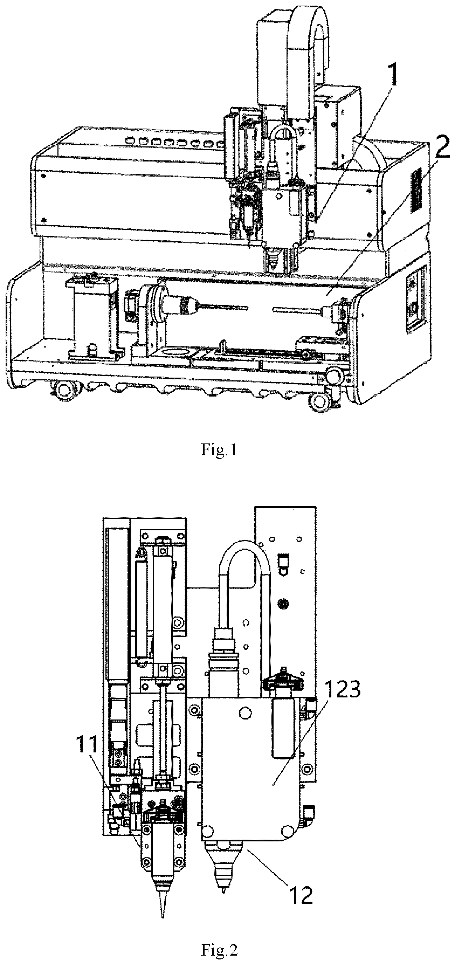

The details of the manufacturing method of the lumen tissue construct of the embodiment of the present disclosure, the lumen tissue construct printing device or the biological construct printing device implementing the manufacturing method, and the working principles thereof will be described below with reference to Figs.1 to 28.

The manufacturing method of the lumen tissue construct of the present disclosure can be implemented by the lumen tissue construct printing device. The manufacturing method may include:
in step 1, installing elastic film A on the support (preferably a rotating rod 211);
in step 3, printing a biological construct on the elastic film A through a bio-block injector 12, specifically as follows: providing a bioprinting unit C through a bio-block container 125, forming a printing unit bonding layer B on a predetermined area of the elastic film A, forming a printing unit bonding layer B by outputting a first reagent via a medical adhesive injector 11 (or a reagent injector; and then printing a bioprinting unit C on the printing unit bonding layer B through the bio-block injector 12 and bonding the bioprinting unit C to the printing unit bonding layer B, thereby forming a biological construct or an intermediate structure of the biological construct;
in step 5, printing a bonding agent (such as a medical adhesive) on the biological construct via the medical adhesive injector 11 to form a construct bonding layer (such as a medical bonding layer);
in step 7, installing the lumen tissue outside a hollow rod 231 in a sleeving way; installing the hollow rod 231 outside the biological construct formed provided with the construct bonding layer; retaining the lumen tissue outside the biological construct while pulling out the hollow rod 231; expanding the elastic film to increase the outer diameter of the biological construct and the outer diameter of the construct bonding layer so that the construct bonding layer is attached to the inner wall of the lumen tissue to make the lumen tissue be bonded to the construct bonding layer.

The detailed structure of the exemplary embodiment of the lumen tissue construct printing device will be described below with reference to Figs. 1-19.

As shown in Fig.1, the lumen tissue construct printing device includes a nozzle assembly 1 and a bioprinting platform 2, and the nozzle assembly 1 prints the biological construct on the inner surface of the lumen tissue through the bioprinting platform 2.

In this exemplary embodiment, by disposing the nozzle assembly 1 and the bioprinting platform 2, the nozzle assembly 1 prints the biological construct on the inner surface of the lumen tissue through the bioprinting platform 2, thereby helping to avoid occurrence of problems such as recurrence of thrombosis and restenosis of the lumen after the lumen tissue is implanted for a long time, and improving the biological reliability of the lumen tissue.

Wherein, the lumen tissue is especially an artificial blood vessel, such as a commercially available Gore blood vessel, and the biological construct is printed on the inner surface of the artificial blood vessel, thereby helping to avoid occurrence of thrombus after the artificial blood vessel is implanted for a long time.

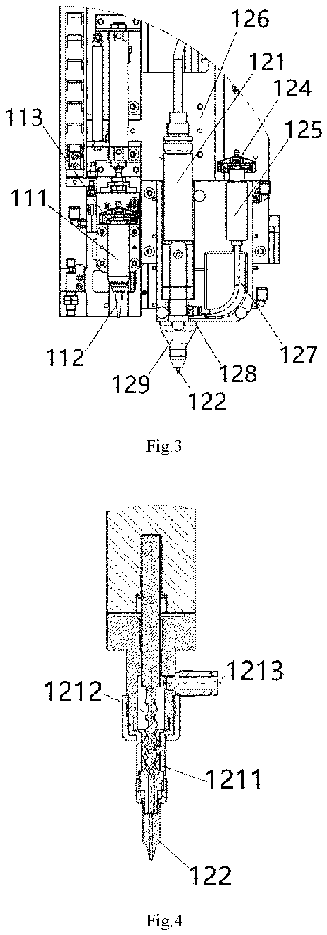

In some embodiments, step 5 includes printing medical adhesive on the biological construct to form the construct bonding layer. Therefore, in an improved embodiment of the lumen tissue construct printing device of the present disclosure, as shown in Fig.2 and Fig.3, the nozzle assembly 1 includes a medical adhesive injector 11. The medical adhesive injector 11 includes a medical adhesive container 111 and a medical adhesive nozzle 112. The medical adhesive container 111 is used to contain the medical adhesive, and the medical adhesive nozzle 112 is directly connected with the medical adhesive container 111. The top of the medical adhesive container 111 is connected with an air pump through an air channel, and a vacuum generator is disposed in the air channel and is used for generating negative pressure in the medical adhesive container 111 in a non-printing state. Since the medical adhesive has excellent fluidity, in the non-printing state, due to the effect of gravity, the medical adhesive will drip slowly, so by additionally providing a vacuum generator in the air channel, certain negative pressure exists in the air channel, the negative pressure counteracts the gravity, so that the medical adhesive no longer drips freely. In some embodiments, as shown in Fig.3, a medical adhesive piston 113 is disposed on the top of the medical adhesive container 111; the medical adhesive piston 113 is connected with the air pump through an air channel; the air pump implements pressurization to squeeze the medical adhesive out of an ink sac; and the vacuum generator is located between the air pump and the medical adhesive piston 113.

In an improved embodiment of the lumen tissue construct printing device of the present disclosure, as shown in Figs.2-4, the nozzle assembly 1 includes a bio-block injector 12. The bio-block injector 12 includes a screw pump 121, a bio-block nozzle 122, and a bio-block container 125. The bio-block container 125 is used to contain biological ink. The biological ink includes a bioprinting unit C, which is, for example, a bio-block. The outlet at the bottom of the bio-block container 125 communicates with a bio-block inlet 128 of the screw pump 121 through a connecting pipe 127 and an inlet connecting part 1213. A bio-block piston 124 is disposed at the top of the bio-block container 125. The bio-block piston 124 is connected with an air pump through an air channel, and the air pump implements pressurization to squeeze the biological ink out of the bio-block container 125 and into the screw pump 121. The screw pump 121 includes a screw stator 1211 and a screw rotor 1212 for squeezing the bio-block entering the screw pump 121 into the bio-block nozzle 122. The screw stator 1211 may be made from a silica gel material.

Due to the physical properties of the bio-blocks, when the outlet of bio-block container 125 is relatively small, the bio-blocks cannot be squeezed out and will be accumulated at the outlet; even if the pressure is increased, the bio-blocks cannot be squeezed out even after being crushed; similarly, the bio-blocks cannot be squeezed out even after taking measures such as angle designing and the like at the exit of the bio-block container 125. However, the demand for printing restricts that the bio-blocks cannot be squeezed out in a large quantity, and only a few bio-blocks can be squeezed out at a time, so the bio-blocks can be sent from the bio-block container 125 to the screw pump 121 and squeezed out by the screw pump 121; since the outlet of the screw pump 121 is relatively large, the quantity of the bio-blocks squeezed out each time is still greater than that of the usage remand, and therefore, a bio-block nozzle 122 is disposed at the outlet of the screw pump 121.

As shown in Fig.4, the screw stator 1211 cannot rotate, and the screw rotor 1212 rotates relative to the screw stator 1211; a groove on the screw rotor 1212 forms a cavity, each cavity is capable of accommodating only a small amount of bio-blocks, and the bio-blocks are sent out to the bio-block nozzle 122 with rotation of the screw rotor 1212. Due to the physical properties of the present biological ink materials, printing needs to be performed at a low temperatures (4 °C); screw stators in existing screw pumps all use rubber materials; the rubber materials will be rapidly aged at a low temperature and black powder will appear during printing, and in the present disclosure, the screw stator 1211 is made from silica gel instead, which is beneficial to avoid appearance of black powder during printing.

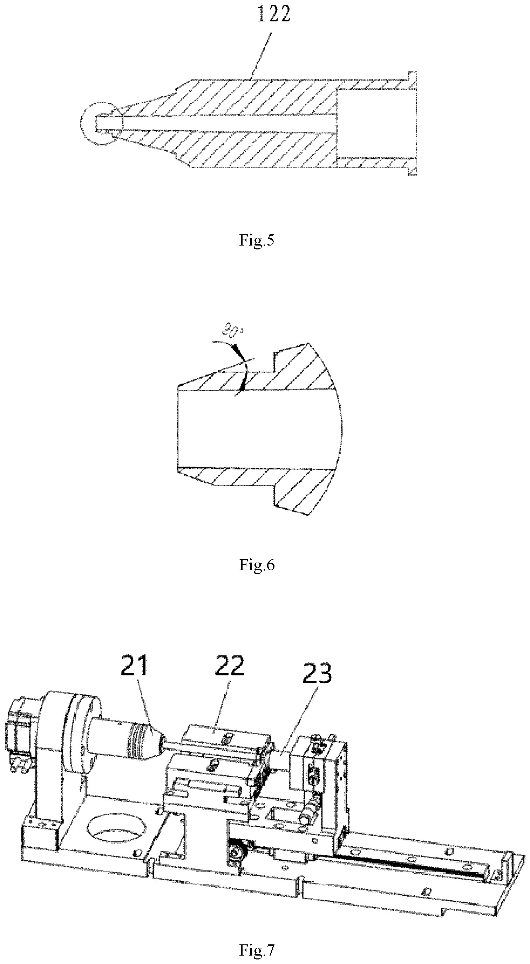

In order to avoid the phenomenon of "hanging beads" at the outlet of the front end of the bio-block nozzle 122 (the circled part in Fig.5) when the bio-block is printed (that is, the biological ink has a relatively high viscosity, the bio-block will not drip directly after being squeezed out and is hung at the outlet of the nozzle, when the subsequent bio-blocks are squeezed out, the previous bio-blocks that have not dripped are piled up with the subsequent ones to form a large droplet hung at the outlet of the nozzle, and this large droplet will drip only when the gravity of this large droplet is greater than the frictional force). In an improved embodiment, on the one hand, as shown in Figs.5-6, the printing outlet end of the bio-block nozzle 122 has a chamfer, and the included angle between the chamfer surface of the chamfer and the center line of the printing outlet of the bio-block nozzle 122 is 10° to 30°. In some embodiments, this included angle, for example, may be 20°. Such design helps to effectively avoid the phenomenon of "hanging beads". On the other hand, the roughness Ra of the outer surface of the printing outlet end of the bio-block nozzle 122 is smaller than or equal to 0.4, the surface smoothness can be increased by plating a coating/polishing the outer surface of the bio-block nozzle 122, thereby helping to better avoid the phenomenon of "hanging beads".

Since the currently used biological ink will tend to be solidified at a temperature greater than 4 °C, it is necessary to maintain the bio-block injector at an ambient temperature of 4 °C; in some embodiments, as shown in Fig.3, the bio-block injector 12 further includes a semiconductor cooling plate 126 located behind the screw pump 121 and the bio-block container 125; and the semiconductor cooling plate 126 is capable of cooling the bio-block injector 12 by heat transfer. In some embodiments, as shown in Fig.3, a thermal insulation sleeve 129 is disposed outside the bio-block nozzle 122, and a certain gap exists between the thermal insulation sleeve 129 and the exterior of the bio-block nozzle 122 to form an air thermal insulation layer. In some embodiments, as shown in Fig.2, the screw pump 121 and the bio-block container 125 are externally sleeved with a heat insulating shell 123, and the heat insulating shell 123 includes a box cover and heat insulating cotton covering the outer surface of the box cover. The heat insulating cotton is beneficial to improve the thermal insulation effect and reduce the heat exchange between the injector and the environment. The box cover is provided with a transparent window for observing the accommodation condition of the biological ink in the bio-block container 125.

In some embodiments, the lumen tissue construct printing device further includes a displacement assembly for moving the nozzle assembly 1, the overall nozzle assembly 1 (the bio-block injector 12 and the medical adhesive injector 11) can be displaced in the vertical direction and the horizontal direction, and the medical adhesive injector 11 can be lifted up and down independently. When the nozzle assembly 1 is in an initial state, the horizontal position of the outlet of the bio-block injector 12 is below the horizontal position of the outlet of the medical adhesive injector 11; when printing, after the displacement assembly lifts down the nozzle assembly 1 to a certain height, the bio-block injector 12 squeezes out the bio-block; and after lumen tissue printing is completed, the medical adhesive injector 11 is separately lifted down after a certain distance of overall lift-up so as to perform medical adhesive printing.

In some embodiments, in order to form a printing unit bonding layer B using a first reagent on the predetermined area of the elastic film A, the medical adhesive injector 11 may be used to form the printing unit bonding layer B. The material (the first reagent) forming the printing unit bonding layer B and the material forming the construct bonding layer may be the same material (for example, both are bonding agents such as medical adhesive), or may be different materials. When the two materials are different, a reagent storage device may be provided for storing the first reagent; when forming the printing unit bonding layer B, the medical adhesive injector 11 may be switched to be connected with the reagent storage device; and the medical adhesive injector 11 is used to print the first reagent to form the printing unit bonding layer B.

In some embodiments, in order to form the printing unit bonding layer B using the first reagent on the predetermined area of the elastic film A, the lumen tissue construct printing device may further be provided with a reagent storage device for storing the first reagent and a reagent injector communicating with the reagent storage device, and the first reagent in the reagent storage device may be distributed on the predetermined area of the elastic film A by means of spraying, printing and the like through the reagent injector so as to form the printing unit bonding layer B. The structure form and movement mode of the reagent injector may refer to the structure form and movement mode of the medical adhesive injector 11.

Wherein, the first reagent may contain a substance having viscosity, or a second reagent may be attached to the surface of the bioprinting unit C, and the first reagent and the second reagent themselves do not contain a viscous substance, but generate mutual bonding force properties through mutual reaction. The first reagent will be explained more detailedly in the following description.

In some embodiments, the step 7 includes:
installing the lumen tissue outside a hollow rod in a sleeving way;
installing the hollow rod outside the biological construct provided with the construct bonding layer in a sleeving way;
retaining the lumen tissue outside the biological construct while pulling out the hollow rod; and
bonding the lumen tissue to the construct bonding layer.

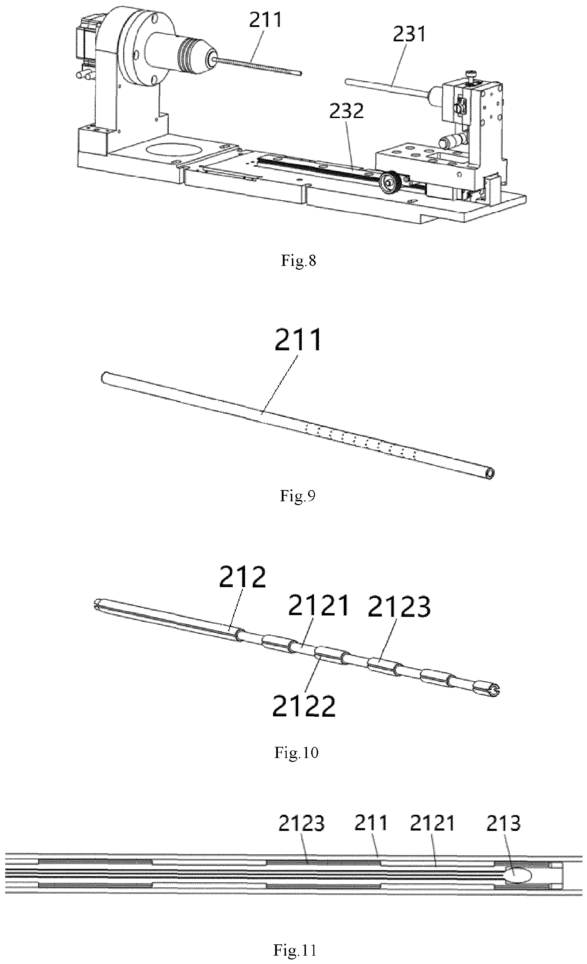

Therefore, in an improved embodiment of the lumen tissue construct printing device of the present disclosure, as shown in Fig.7 and Fig.8, the bioprinting platform 2 includes a platform base, a rotating portion 21, and a matching portion 23 that is capable of moving relative to the rotating portion 21; the rotating portion 21 includes a rotating rod 211; the rotating rod 211 serves as a support for printing a biological construct and is used to bear the bio-blocks and the medical adhesive to form a biological construct, and the matching portion 23 includes a hollow rod 231 and a displacement mechanism 232; the outer wall of the hollow rod 231 is used to bear the lumen tissue; and an inner cavity is used to accommodate the rotating rod 211 and the biological construct.

After the biological construct is prepared on the rotating rod 211, the hollow rod 231 is displaced toward the rotating portion 21 through the displacement mechanism 232, the rotating rod 211 and the biological construct enter the inner cavity of the hollow rod 231, and the lumen tissue sleeving the exterior of the hollow rod 231 is displaced out of the biological construct with the hollow rod 231; in some embodiments, the surface of the hollow rod 231 is plated with a Teflon layer capable of helping to avoid reaction between the medical adhesive and a metal surface. Then the hollow rod 231 is displaced toward the opposite direction through the displacement mechanism 232, the lumen tissue is removed from the hollow rod 231 and is then disposed on the outer surface of the biological construct in a sleeving way to complete the assembly to obtain the lumen tissue construct.

The outer wall of the rotating rod 211 is covered with the elastic film; when performing biological construct printing, the elastic film is in a natural state, namely it covers the surface of the rotating rod 211; and the bio-block forms the biological construct on the surface of the elastic film, thereby helping to remove the biological construct.

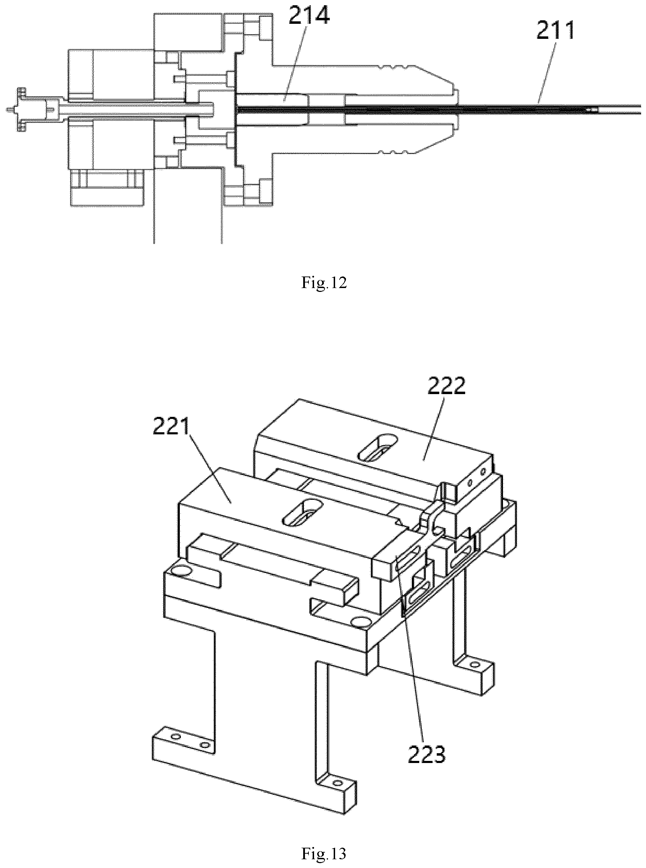

In some embodiments, as shown in Fig.9, the interior of the rotating rod 211 is hollow, and the outer wall of the rotating rod 211 is provided with a vent hole communicating with the interior in order to exhaust the air inside the rotating rod 211 to prop up the elastic film A; when the biological construct and the lumen tissue are assembled, gas is introduced to the rotating rod 211, the gas expands outward via the air outlet to prop the elastic film A (may be imaged as that a balloon is inflated), the biological construct on the surface of the elastic film A is displaced outward with expansion of the elastic film, and finally makes contact with the inner wall of the lumen tissue and is bonded to the inner wall of the lumen tissue to obtain the lumen tissue construct. Practice shows that this implementation mode is simple to operate and easy to implement, and has relatively high implementability. In some embodiments, as shown in Fig.12, a sealing ring 214 is disposed inside the rotating part 21, the rotating rod 211 is detachably connected with the sealing ring 214, the function of the sealing ring 214 is to seal the inner cavity of the rotating rod 211, so that the process of inflating the elastic film A is more controllable.

In some embodiments, printing the biological construct on the elastic film A further includes heating the support. As shown in Fig.10 and Fig.11, a heating element 212 is further disposed inside the rotating rod 211. The heating element 212 can accelerate the solidification of the bioprinting unit and the printing unit bonding layer B and shorten the preparation time of the biological construct, and the heating element 212 can maintain the surface temperature of the rotating rod at 37 °C to 38 °C. In some embodiments, as shown in Fig.11, the end, close to the matching portion 23, of the heating element 212 is provided with a temperature detection element 213 for detecting the temperature of the heating element 212 so as to maintain the surface temperature of the rotating rod 211 in real time.

In a specific or improved embodiment, as shown in Fig.10 and Fig.11, the heating element 212 includes heating sections 2121 and partition sections 2123 that are disposed alternatively. A connecting groove 2122 is formed in the surface of the partition section 2123. A resistance wire is wound on the surface of the heating section 2121, and no resistance wire is wound on the surface of the partition section 2123. The resistance wire between adjacent heating sections 2121 passes through the connecting groove 2122. The diameter of the heating section 2121 is smaller than that of the partition section 2123. The heating element 212 and the rotating rod 211 are in clearance fit, and the outer wall of the partition section 2123 is in contact with the inner wall of the rotating rod 211. The purpose of setting the partition section is to protect the resistance wire by sleeving the heating element 212 inside the rotating rod 211, thereby helping to avoid damages of the resistance wire during assembly.

Regarding to how to remove the lumen tissue from the hollow rod 231, in some embodiments, as shown in Fig.7, the bioprinting platform 2 further includes a clamping mechanism 22 for clamping the lumen tissue when the hollow rod 231 is displaced in the opposite direction so that the lumen tissue is removed from the hollow rod and is connected with the biological construct in a sleeving way, and this implementation mode is easy to implement and has relatively high reliability.

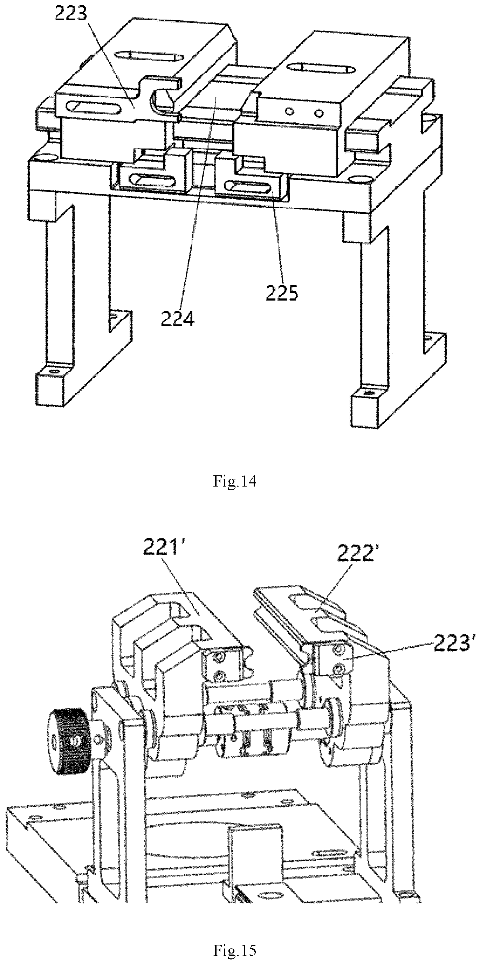

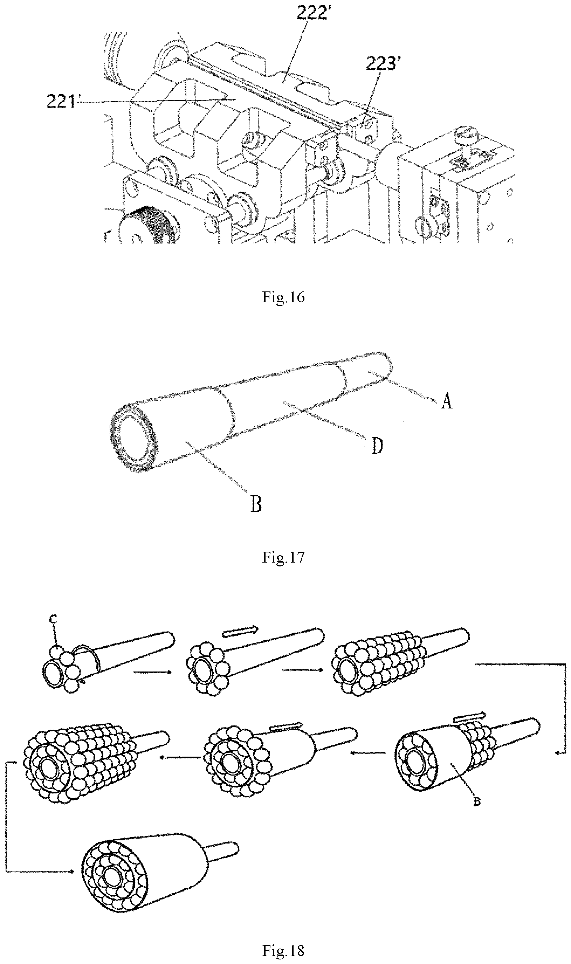

In some embodiments, as shown in Figs. 13-16, the clamping mechanism 22 includes first clamping blocks 221, 221' and second clamping blocks 222, 222' that can move relatively. As shown in Fig.13 and Fig.14, the relative movement of the first clamping block 221 and the second clamping block 222 can be achieved by providing a guide rail; as shown in Fig.15 and Fig.16, the relative movement of the first clamping block 221' and the second clamping block 222' can also be achieved by providing a screw nut mechanism. In some embodiments, as shown in Fig.14, the clamping mechanism 22 further includes limit blocks 225 that are disposed at the bottom of the first clamping block 221 and the bottom of the second clamping block 222 in order to limit the relative movement of the first clamping block 221 and the second clamping block 222, so that the first clamping block 221 and the second clamping block 222 are tangent to the outer wall of the lumen tissue, and a certain limitation effect on the lumen tissue can be achieved in the process of assembling the lumen tissue construct.

In order to guarantee that the lumen tissue is removed from the hollow rod 231 as much as possible, in one embodiment, as shown in Figs. 13-16, the clamping mechanism 22 further includes retaining members 223, 223' for acting on the tail end of the lumen tissue when the hollow rod 231 is displaced in the opposite direction so that the lumen tissue is removed from the hollow rod 231, thereby preventing the lumen tissue from following the hollow rod 231. The retaining members 223, 223' may be disposed at the tail ends of the first clamping block 221, 221' and/or the second clamping block 222, 222', or may be directly disposed on the clamping mechanism in a manner of being independent of the first clamping block and the second clamping block. In some embodiments, the retaining member 223 is cooperatively provided with a retaining ring acting at the tail end of the lumen tissue, so that the lumen tissue is more easily removed from the hollow rod 231. In some embodiments, as shown in Fig.14, the clamping mechanism 22 further includes support platforms 224 disposed at the bottom of the first clamping block 221 and the bottom of the second clamping block 222 in order to support the lumen tissue. In the process of matching the lumen tissue with the biological construct, the support platform 224 is exactly tangent to the outer wall of the bottom of the lumen tissue to provide upward force for the lumen tissue, thereby helping to avoid the fall of the lumen tissue.

In one embodiment of the lumen tissue construct printing device of the present disclosure, the lumen tissue construct printing device further includes a liquid reservoir disposed below the rotating rod 211 in order to receive the lumen tissue construct that is removed and falls from the clamping mechanism 22. After printing and assembly is completed, the lumen tissue construct is held by the clamping mechanism 22, the overall rotating rod 211 is withdrawn in the opposite direction, the lumen tissue construct is located over the liquid reservoir and is supported by the clamping mechanism 22; at the moment, the clamping mechanism 22 withdraws the clamping force, and the lumen tissue construct falls vertically into the liquid reservoir. Such design is beneficial to avoid the introduction of new pollution during a transfer operation process implemented manually, by mechanical arms and the like after printing and assembly is completed, or to avoid damages and the like of the inner wall of the printed blood vessel due to improper operation during the operation process, and is convenient for the packaging of finished products, etc.

The embodiment of the present disclosure implements the manufacturing method of the lumen tissue construct by utilizing the above-mentioned lumen tissue construct printing device. In the manufacturing method, printing the biological construct includes a film coating step of coating the outer wall of the rotating rod 211 with a layer of elastic film A before printing the biological construct. When the biological construct is printed, the elastic film A is in a natural state, namely coats on the surface of the rotating rod 211, and the bioprinting unit C forms a biological construct on the surface of the elastic film A, thereby facilitating the removal of the biological construct.

In some embodiments, bonding the lumen tissue to the construct bonding layer includes: expanding the elastic film to increase the outer diameter of the biological construct and the outer diameter of the construct bonding layer to make sure that the construct bonding layer is attached to the inner wall of the lumen tissue, namely a film expanding step. In some embodiments, gas is delivered from inside the support to the surface of the support to expand the elastic film.

After the lumen tissue is disposed outside the biological construct in a sleeving way, gas is introduced to the elastic film to prop up the elastic film, so that the biological construct is attached to the inner wall of the lumen tissue. The biological construct on the surface of the elastic film is displaced outwards with the expansion of the elastic film, finally makes contact with the inner wall of the lumen tissue and is bonded to the inner wall of the lumen tissue, so that the biological construct is completely evenly, completely and smoothly attached to the inner wall of the lumen tissue to obtain a lumen tissue construct, such as an artificial tissue precursor. Practice shows that the implementation method is simple to operate and easy to implement, and has relatively high implementability.

The process of preparing the lumen tissue construct using the lumen tissue construct printing device of the present disclosure will be described by taking the embodiments shown in Figs. 1-16 as an example as follows:
in step 1, installing the elastic film A on the rotating rod 211 in a sleeving way;
in step 3, printing a first reagent using the medical adhesive injector 11 (or the reagent injector) on the predetermined area of the elastic film A to form the printing unit bonding layer B; printing the bioprinting unit C provided by the bio-block container 125 on the printing unit bonding layer B through the bio-block injector 12 and bonding the bioprinting unit C to the printing unit bonding layer B; if only a biological construct with a single layer of bioprinting unit C is needed, the structure formed at this moment is the biological construct itself; if a biological construct with a plurality of layers of bioprinting unit C is needed, the structure formed at this moment is the intermediate structure of the biological construct; after that, further forming a printing unit bonding layer B on the intermediate structure obtained in the previous step to obtain an intermediate structure including the printing unit bonding layer B, subsequently bonding the bioprinting unit C to the intermediate structure including the printing unit bonding layer B; when necessary, repeating the steps of forming the printing unit bonding layer B on the intermediate structure and subsequently bonding the bioprinting unit C to the printing unit bonding layer B one or more times to form a biological construct with a plurality of bioprinting bonding layers C.

In step 5, a medical adhesive layer for bonding the bio-block and the lumen tissue is evenly squeezed out on the surface of the biological construct through the medical adhesive injector 11;
in step 7, after the biological construct is prepared, the hollow rod 231 moves toward the rotating rod 211 until the hollow rod 231 completely sleeves the exterior of the rotating rod 211, at the moment the lumen tissue is completely located outside the biological construct, the hollow rod 231 moves toward the direction away from the rotating rod 211, at the moment the clamping mechanism 22 prevents the lumen tissue from moving with the hollow rod 231, and finally, the hollow rod 231 is completely separated from the rotating rod 211, but the lumen tissue remains outside the biological construct. Due to the limitation of a mechanical structure, at the moment there must be a gap between the lumen tissue and the biological construct; at the moment, a support platform 224 provides an upward force to the lumen tissue, thereby helping to avoid uneven attachment between the biological construct and the artificial blood vessel caused by downward movement of the lumen tissue due to the effect of gravity. Then, gas is introduced to the rotating rod 211 to prop up the elastic film, so that the biological construct is completely attached to the inner wall of the lumen tissue. The heating element 212 implements heating to accelerate the solidification of the bioprinting unit and the printing unit bonding layer B to finally obtain a lumen tissue construct, and the lumen tissue construct can be just removed from the rotating rod 211.

When the biological construct is printed, the elastic film A coats the surface of the rotating rod in a natural state; the bioprinting unit forms a biological construct on the surface of the elastic film A; after the preparation of the lumen tissue construct is completed, since the elastic film A coats the surface of the rotating rod in a natural state, at the moment the inner diameter of the elastic film A is smaller than that of the lumen tissue construct, so the lumen tissue construct can be removed conveniently.

In some embodiments, the manufacturing method of the lumen tissue construct further includes detecting the flatness of the inner wall of the biological construct. Since the flatness of the inner wall of the printed lumen tissue construct needs to be detected, in one embodiment of the lumen tissue construct printing device of the present disclosure, the lumen tissue construct printing device further includes an optical probe which can move inside the rotating rod 211 and is used to detect the flatness of the inner wall of the biological structure, and the rotating rod 211 is made from a transparent material. The optical probe is designed in a form of being movable inside the rotating rod 211; before removing the lumen tissue construct from the rotating rod 211, the optical probe is moved to take a picture of the inner wall of the biological construct through image acquisition software to determine whether a printed bio-block coating is complete and smooth or not, a hollow structure inside the rotating rod 211 is fully utilized to increase the structural utilization rate, and relatively high implementability is achieved.

For how to realize that the optical probe can be moved inside the rotating rod 211, in some embodiments, the optical probe is fixedly disposed inside the hollow rod 231, for example, the hollow rod 231 is designed as a double-layer nested structure, the first layer is used to sleeve an artificial blood vessel, and the optical probe is disposed at the front end of the second layer; the rotating rod 211 is also of a double-layer structure, gas is introduced to the interlayer in the double-layer structure to prop up the elastic film, and the elastic film only covers the surface of the rotating rod 211 without covering the front end, thus allowing the optical probe to extend into the rotating rod 211. During the assembly process, the lumen tissue is disposed on the surface of the biological construct in a sleeving way, the optical probe also moves with the hollow rod 231 to the farthest end of the biological construct; when the lumen tissue is removed, the optical probe also moves with the hollow rod 231 to the forefront of an artificial precursor tissue, thereby completing flatness detection during the assembly process. Certainly, in some other improved embodiments, the optical probe is movably disposed inside the hollow rod 231, namely the optical probe independently moves relative to the hollow rod 231, and flatness detection can be completed as well. In some other improved embodiments, the optical probe is movably disposed inside the rotating rod 211, and the optical probe moves from one end to the other end inside the rotating rod 211 to complete the flatness detection.

Although the lumen tissue construct printing device shown in Figs. 1-16 is adopted to print the biological construct in the above-mentioned embodiments of the present disclosure, the term "bioprinting" used herein refers to: printing utilizing biological materials (including but not limited to, biomolecules such as proteins, lipids, nucleic acids and metabolites; cells such as cell solutions, cell-containing gels, cell suspensions, cell concentrates, multicellular aggregates and multicellular bodies; subcellular structures such as organelles and cell membranes; and molecules related to biomolecules, such as synthetic biomolecules or analogs of biomolecules). As used herein, the term "printing" refers to a process of depositing a material according to a predetermined pattern. In the present disclosure, bioprinting is achieved by a method that is matched with an automatic or semi-automatic computer-assisted three-dimensional prototype device (such as a bioprinter). However, in the present disclosure, "printing" (for example, bioprinting) can be performed by various methods, including but not limited to, printing performed using a printer (for example, a 3D printer or a bioprinter); printing performed using automated or non-automated mechanical processes (not a printer); and printing performed by manual placement or manual deposition (for example, using a pipette).

Another two embodiments of the preparation of the biological construct are described below with reference to Figs.17-28, the first one is to prepare the biological construct by manually grabbing-placing the bioprinting unit, and the second one is to prepare the biological construct by printing of the biological construct printing device.

In one embodiment of the printing of the biological construct of the present disclosure, as shown in Figs. 17-19, in the manufacturing method of the lumen tissue construct, step 3 (a biological construct printing step) includes:
step 31: providing at least one bioprinting unit C;
step 33: forming a printing unit bonding layer B on the predetermined area of the elastic film A;
step 35: bonding the bioprinting unit C to the printing unit bonding layer B to form a biological construct or an intermediate structure of the biological construct, namely at least forming a part of the biological construct.

The printing unit bonding layer B can be formed by means of coating, printing a bonding agent, etc.

In the schematic embodiment shown in Fig.17, the biological construct is a lumen structure, and the support is a rotating rod. When the biological construct is printed, the predetermined area of the elastic film A is coated with the first reagent to form the printing unit bonding layer B, and then the bioprinting unit C is bonded to the printing unit bonding layer B, namely before the bioprinting unit C is printed on the elastic film A, the predetermined area of the elastic film A has been coated with the printing unit bonding layer B that the bioprinting unit C can be bonded to, thereby realizing existence of the printing unit bonding layer B between the elastic film A and the bioprinting unit C, improving the situation that a part of the area of the bioprinting unit is not coated with the bonding agent in an existing method in which the bioprinting unit and the bonding agent are together printed, and helping to avoid the condition that a part of the bioprinting unit slips or falls on the support.

Compared to a scheme of mixed squeezing adopting a biocompatible material with a relatively high viscosity and a bioprinting unit, the biological construct printing step achieves independent printing and arrangement of the printing unit bonding layer B and the bioprinting unit C separately, thereby helping to avoid the problems about poor fluidity, discontinuous discharge and easy blocking of the outlet when the mixed material is squeezed out, helping to avoid the problem about relatively large damage of cells in biological ink when overcoming the mechanical external force of the injector to realize printing of the bioprinting unit with a relatively high viscosity during the printing process.

In the biological construct printing step, the bioprinting unit is bonded to the support through a bonding layer without providing a needle for fixing the cell body of the bioprinting unit, thereby helping to avoid damages caused by puncture of the needle to the cell body in the bioprinting unit, which is beneficial to maintain the biological activity of the bioprinting unit. Moreover, the embodiments of the present disclosure have low requirements on the uniformity of the bioprinting unit, as long as the bioprinting unit is bonded to the bonding layer, and the spacing between the print units can be set as needed during the printing process, thereby helping to avoid the adjacent bioprinting units from squeezing each other. In addition, a user can change the distribution condition of the bioprinting unit at any time according to actual situation, which is beneficial to increase the printing freedom degree and flexibility of the biological construct, and form a variety of biological constructs by combination of bioprinting units which are different in particle size.

Regarding to how the bioprinting unit C can be bonded to the printing unit bonding layer B, in some embodiments, the first reagent contains a substance which is viscous, the substance can alone generate bonding force, the bioprinting unit C is bonded to the printing unit bonding layer B by utilizing the viscosity of the first reagent. As another implementation mode of the biological construct printing step, a second reagent is attached to all or a part of the surface of the bioprinting unit C, and the bioprinting unit C is bonded to the printing unit bonding layer B by virtue of the bonding effect generated by contact of the first reagent and the second reagent. The bonding effect generated by the contact between the first reagent and the second reagent is relatively good to bond the bioprinting unit C to the printing unit bonding layer B quite reliably and stably, and relatively high implementability is achieved. For example, the first reagent contains thrombin, the second reagent contains brinase, and the thrombin and the brinase can quickly react with each other when the first reagent and the second reagent are in contact with each other, thereby achieving bonding and fixation of the bioprinting unit C. Optionally, the first reagent contains brinase and the second reagent contains thrombin, resulting in a bonding effect as well. For another example, the first reagent contains calcium chloride, the second reagent contains sodium alginate, and the calcium chloride and the sodium alginate can quickly react with each other when the first reagent and the second reagent are in contact with each other, thereby achieving bonding and fixation of the bioprinting unit C, in a similar way. Optionally, the first reagent contains sodium alginate and the second reagent contains calcium chloride, resulting in a bonding effect as well. In these implementation modes, the first reagent and the second reagent themselves do not contain a viscous substance, but instead generate mutual bonding force through mutual reaction; compared with the method of using a substance which is viscous, a better bonding effect is achieved, and independent operation is also facilitated.

In some embodiments, step 33 includes: forming an isolation medium layer D on the predetermined area of the elastic film A, and then forming the printing unit bonding layer B on the elastic film A through the isolation medium layer D. The purpose of forming the isolation medium layer D is to facilitate the detachment of the overall biological construct from the elastic film A after printing is completed. The isolation medium layer D can be formed by means of coating and printing an isolation medium.

In some embodiments, the isolation medium includes a temperature-sensitive hydrogel. On the one hand, by virtue of the temperature sensitivity of the temperature-sensitive hydrogel, the printed biological construct can be easily and totally detached from the elastic film A; and on the other hand, the temperature-sensitive hydrogel can conveniently adsorb the printing unit bonding layer B and the bioprinting unit C on the elastic film A to guarantee that the bioprinting unit C is not detached.

In the process of printing a biological construct, it is necessary to print a plurality of layers of bioprinting units C; in some embodiments, as shown in Figs.18-19, printing the biological construct on the elastic film A further includes:
step 37: forming a printing unit bonding layer B on the intermediate structure obtained in the previous step to obtain an intermediate structure including the printing unit bonding layer B;
step 39: bonding the bioprinting unit C to the obtained intermediate structure including the printing unit bonding layer B.

In some embodiments, printing the biological construct on the elastic film A further includes repeating step 37 and step 39 one or more times.

In this embodiment, the biological construct structure obtained in the previous step is coated with the first reagent for bonding the next layer of bioprinting unit C to be printed, and then the bioprinting unit C is bonded and fixed by a new bonding layer formed by the coated first reagent. Repeating this once or plurality of times to obtain a predetermined number of layers of biological constructs.

In order to protect or support the printed and shaped biological construct, in some embodiments, step 5 includes: forming a support layer on the surface of the biological construct; and then forming a construct bonding layer on the biological construct through the support layer. The support layer, for example, can be formed by spraying and printing a molding material or a support material.

In the above-mentioned embodiments, the biological construct is a lumen-like structure, and the support is a rotating rod 211 in some embodiments. This biological construct printing step is particularly suitable for printing lumen-like structures. The biological construct printing step can prevent the printed bioprinting unit from slipping or falling when the rotating rod rotates, thereby better solving the problem of how to realize precise printing and arrangement.

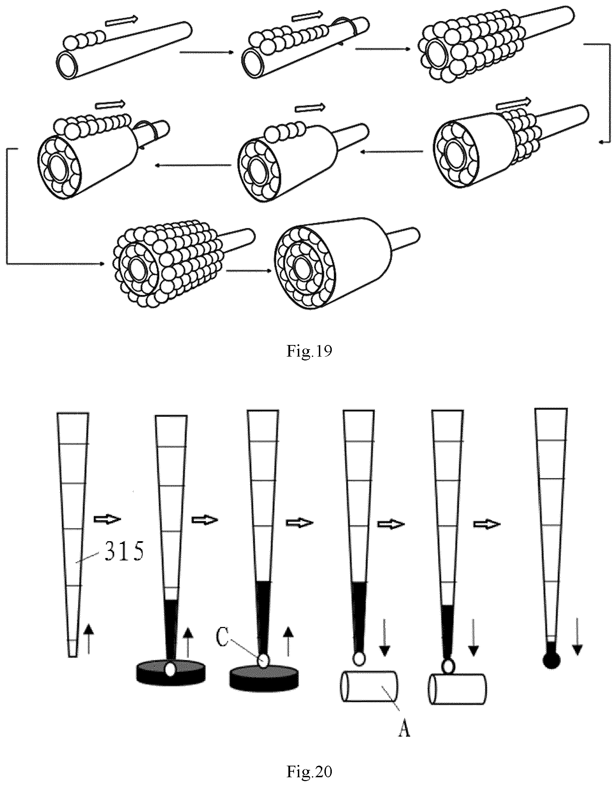

The lumen-like structure is, for example, a blood vessel precursor; the molding material or the support material includes a biocompatible material; and the biocompatible material is, for example, an artificial blood vessel material. As shown in Fig.18 and Fig.19, in the printing process using the biological construct printing step, for the sequential arrangement way of the bioprinting units C, a "circumferential arrays first, then linear arrays" arrangement way shown in Fig.18 can be selected, or a "linear arrays first, then circumferential arrays" arrangement way shown in Fig.19 can also be selected.

For how to prepare the biological construct, a continuous printing method can be selected, namely a printing injector (such as a bio-block injector 12) is used to continuously print and spray the bioprinting unit C on the printing unit bonding layer, or other forms can also be adopted. For example, a grabbing-placing printing form shown in Fig.20 can be adopted. The grabbing-placing printing form shown in Fig.20 can be applied to a manual mode and can also be applied to a biological construct printing device.

In the embodiment shown in Fig.20, when adopting a manual mode, step 3 (a biological construct printing step) includes:
step 31: providing at least one bioprinting unit C in the bio-block container;
step 33: forming a printing unit bonding layer B on the predetermined area of the elastic film A that is installed on the rotating rod in a sleeving way;
step 35: manually sucking the bioprinting unit C from the bio-block container by using a suction component 315, moving the suction component 315 to the position of the printing unit bonding layer B and releasing the bioprinting unit C to bond the bioprinting unit C to the printing unit bonding layer B, sucking and bonding the bioprinting unit C a plurality of times to until a layer of bioprinting unit C is formed on the printing unit bonding layer B to form a biological construct or an intermediate structure of the biological construct, namely at least forming a part of a biological construct.

This manual grabbing-placing placement method, on the one hand, can solve the problem of precise printing and arrangement, and the spacing between the printing units can be set as needed during the printing process, thereby helping to avoid mutual extrusion between adjacent bioprinting units. On the other hand, it is beneficial to avoid problems about blocking of the nozzle, uneven arrangement of bio-blocks and the like in the printing process of a traditional printing method. In some embodiments, each suction component 315 sucks only a single bioprinting unit C at a time, so that the precise placement of the bioprinting units C is well achieved, and then the bioprinting units C are placed one by one on the printing unit bonding layer B for bonding, thereby achieving high-accuracy printing and manufacturing of biological constructs.

Regarding to how to achieve the suction and release of the bioprinting units C, in some embodiments, it includes: pre-sucking liquid using the suction component 315 to form liquid film at the suction end of the suction component 315; sucking the bioprinting units C using the suction component 315 to retain the bioprinting units C at the suction end of the suction component 315 and make the bioprinting units C be in contact with the liquid film.

In this embodiment, the liquid pre-sucked into the suction component 315 is capable of wetting the suction end of the suction component 315 (the suction component in this embodiment is a pipette gun, and the suction end is specifically the opening of the pipette gun; in other embodiments, a pipette, a burette, a syringe and the like may also be used), so that liquid film is formed at the suction end of the suction component 315; when the suction component 315 continues to suck the bioprinting unit C, the liquid film is beneficial to avoid direct contact between the suction end and the bioprinting unit C, thereby preventing the shape and biological activity of the bioprinting unit C from being damaged and destroyed due to rigid contact with the suction end. Moreover, since the suction end and the bioprinting unit C can be separated by the liquid film, even a bioprinting unit C with a particle size smaller than the diameter of the suction port of the suction end can also be retained at the suction port under the actions of the liquid film and negative pressure, therefore, the liquid film can also be provided to effectively meet the micro-particle size requirements of a non-suction particle acquisition method for the bioprinting unit C and expand the application range of this non-suction micro-particle acquisition method, so that more specifications and more types of bioprinting units C can be manipulated on the premise of minor damages. And the number of cells contained in the bioprinting units C with a relatively small particle size is relatively small, and the demand for nutrient supply is relatively low, therefore the use of liquid film to achieve non-suction acquisition of the bioprinting units C with a relatively small particle size is also beneficial to better maintain the biological activity of the bioprinting units C during the printing process, and helps to finally obtain a biological construct with better biological activity.

Besides, the liquid film pre-formed at the suction end can achieve an attraction effect on the bio-printing unit C that is sucked later, so that the bioprinting unit C is not only subjected to negative pressure, but is also subjected to the surface tension of the liquid film; on the one hand, this can reduce the negative pressure to be applied to the suction component 315, can reduce the technical requirements of suction, increase the success probability and can also reduce the damages caused by the suction force to the bioprinting unit C; on the other hand, it can also make the bioprinting unit C be more firmly retained at the suction port of the suction end, thus the firmness of the suction process is improved and the bioprinting unit C can be firmly retained at the opening of the pipette during the displacement process and is not prone to falling off, thus the stability and reliability of the displacement process are improved; in another aspect, by virtue of the liquid film, the bioprinting unit C can be kept in a liquid environment all the time from it is sucked out until it is placed in a set printing position, and the liquid environment is an important factor for the bioprinting unit C to maintain the biological activity, therefore, by arranging the liquid film, the bioprinting unit C can still maintain good activity even during the displacement process, and thus the success rate of bioprinting is increased.

In some embodiments, the liquid pre-sucked by the suction component 315 includes a second reagent; the bioprinting unit C makes contact with the liquid film to make sure that the second reagent is attached to all or a part of the bioprinting unit C; and the bioprinting unit C is bonded to the printing unit bonding layer B by the bonding effect generated by contact of the first reagent and the second reagent. By adding the second reagent to the liquid pre-sucked by the suction component 315, the second reagent can be attached to the bioprinting unit C during the suction process, so there is no need to coat all or a part of the surface of the bioprinting unit C with the second reagent before the bioprinting unit C is grabbed, thereby simplifying the printing process and achieving relatively high implementability.

In some embodiments, the suction component 315 is moved to the vicinity of the printing unit bonding layer B, and the suction component 315 is used to release the bioprinting unit C that falls from the suction end of the suction component 315 to the printing unit bonding layer B, thus the bioprinting unit C can fall to the printing unit bonding layer B by utilizing its own weight, thereby reducing damages of the printing unit bonding layer B and the bioprinting unit C caused by contact of the suction end of the suction component 315 and the printing unit bonding layer B.

The manufacturing method of the biological construct of the embodiment of the present disclosure can be realized by using the fore-mentioned lumen tissue construct printing device, can also be realized by utilizing the biological construct printing device, and finally the printed biological construct is assembled with the lumen tissue to form a lumen tissue construct.

In the embodiments shown in Figs. 17-28, step 3 (a biological construct printing step) includes:
step 31: providing at least one bioprinting unit C in the printing unit kit 304;
step 33: coating the predetermined area of the elastic film A sleeved on the support 329 (for example, the rotating rod) with the first reagent by using the first coating component 321 to form the printing unit bonding layer B;
step 35: placing the bioprinting unit C on the printing unit bonding layer B by using a placement mechanism (for example, a printing injector) so that the bioprinting unit C is bonded to the printing unit bonding layer B to form a biological construct or an intermediate structure of the biological construct.

In an exemplary embodiment of the biological construct printing device, as shown in Figs. 17-28, the biological construct printing device includes: a support 329, a first coating component 321 and a placement mechanism, wherein the first coating component 321 can be used to coat the predetermined area of the elastic film A sleeved on the support 329 with the first reagent to form the printing unit bonding layer B; the placement mechanism can be used to place the bioprinting unit C on the printing unit bonding layer B to make sure that the bioprinting unit C is bonded to the printing unit bonding layer B to form a biological construct or an intermediate structure of the biological construct.

In this exemplary embodiment, the support 329, the first coating component 321, and the placement mechanism are provided. The support 329 is used to support the bioprinting unit C; the predetermined area of the elastic film A sleeved on the support 329 is coated with the first reagent through the first coating component 321 to form the printing unit bonding layer B, and then the bioprinting unit C is placed on the printing unit bonding layer B by the placement mechanism, so that the bioprinting unit C is bonded to the printing unit bonding layer B, namely before the bioprinting unit C is printed on the elastic film A, the predetermined area of the elastic film A has been coated with the printing unit bonding layer B to which the bioprinting unit C can be bonded, thereby realizing that there is a printing unit bonding layer B for bonding the bioprinting unit C between the elastic film A and the bioprinting unit C, improving the condition that some areas of the bioprinting unit are not coated with the bonding agent in an existing method in which the bioprinting unit and the bonding agent are printed together, and helping to avoid the condition that a part of the bioprinting unit slips or falls on the elastic film A.

Compared with the scheme of mixed squeezing using a biocompatible material with a relatively high viscosity and a bioprinting unit, the biological construct printing device of the embodiment of the present disclosure can implement separate and independent layered printing and arrangement of the printing unit bonding layer B and the bioprinting unit C, thereby helping to avoid the problems about poor fluidity, discontinuous discharge and easy blocking of the outlet when the mixed material is squeezed out, helping to avoid the problem about relatively large damage of cells in biological ink when overcoming the mechanical external force of the injector to realize printing of the bioprinting unit with a relatively high viscosity during the printing process.

The biological construct printing device of the embodiment of the present disclosure realizes bonding of the bioprinting unit C to the elastic film A through the printing unit bonding layer B, a needle for fixing the cell bodies in the bioprinting unit C needs not to be provided, thereby helping to avoid damages caused by the puncture of the needle to the cell bodies in the bioprinting unit C, and helping to maintain the biological activity of the bioprinting unit C. Moreover, the embodiments of the present disclosure have low requirements on the uniformity of the bioprinting unit, as long as the bioprinting unit C is bonded to the bonding layer, and the spacing between the printing units can be set as needed during the printing process, thereby helping to avoid the adjacent bioprinting units from squeezing each other. In addition, a user can change the distribution condition of the bioprinting unit at any time according to actual condition, which is beneficial to increase the printing freedom degree and flexibility of the biological construct, and form a variety of biological constructs by combination of bioprinting units which are different in particle size.

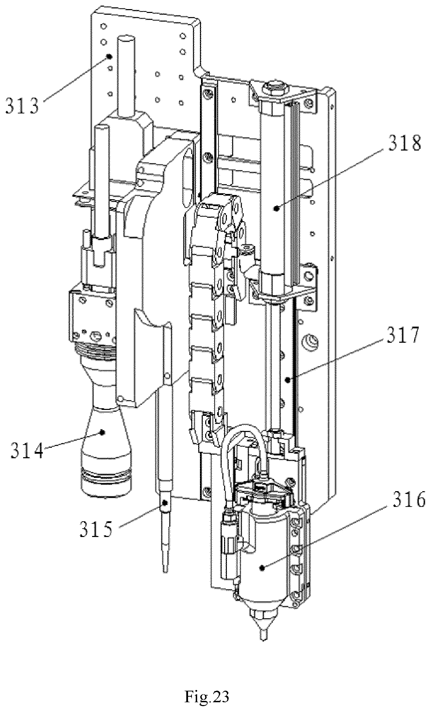

In an improved embodiment of the biological construct printing device of the present disclosure, as shown in Fig.17 and Fig.23, the biological construct printing device further includes a second coating component 316, the second coating component 316 can be used for pre-coating the predetermined area of the elastic film A sleeved on the support 329 with the isolation medium to form an isolation medium layer D, and the printing unit bonding layer B is coated on the elastic film A through the isolation medium layer D. Before coating the printing unit bonding layer B, the predetermined area of the elastic film A sleeved on the support 329 is pre-coated with the isolation medium by a second coating component 316 to form the isolation medium layer D, then the isolation medium layer D is coated with the printing unit bonding layer B by a first coating component 321, and the purpose of coating the isolation medium layer D is to facilitate removal of the overall biological construct from the elastic film A after printing is completed.

In some embodiments, the isolation medium includes temperature-sensitive hydrogel, on the one hand, by virtue of the temperature sensitivity of the temperature-sensitive hydrogel, the printed biological construct can be easily and totally detached from the elastic film A; and on the other hand, the temperature-sensitive hydrogel can conveniently adsorb the printing unit bonding layer B and the bioprinting unit C on the elastic film A to guarantee that the bioprinting unit C is not detached.

Regarding to the structural form of the placement mechanism, the placement mechanism may be a traditional continuous printing injector, and the continuous printing injector realizes spray coating of the bioprinting unit C on the printing unit bonding layer B. A grabbing-placing structural form may be adopted, namely, in some embodiments, the placement mechanism includes a suction component 315 capable of sucking and moving the bioprinting unit C to the position of the printing unit bonding layer B to release the bioprinting unit C, so that the bioprinting unit C is bonded to the printing unit bonding layer B. The suction component 315 can realize a grabbing-placing placement way, on the one hand, can solve the problem of precise printing and arrangement, and the spacing between the printing units can be set as needed during the printing process, thereby helping to avoid adjacent bioprinting units from squeezing each other. On the other hand, it is beneficial to avoid problems about blocking of the injector, uneven arrangement of bio-blocks and the like in the printing process of a traditional printing method. In some embodiments, the suction component 315 includes at least one suction position; only a single bioprinting unit C is sucked at each suction position at a time, so that precise placement of the bioprinting units C is well achieved, and then the bioprinting units C are placed one by one on the printing unit bonding layer B for bonding, thereby achieving high-accuracy printing and manufacturing of the biological constructs. Wherein, the suction component 315 may include one suction position, or may include a plurality of suction positions, and the plurality of suction positions can simultaneously release the sucked plurality of bioprinting units C to the printing unit bonding layer B, thereby helping to improve the printing efficiency and saving the printing time.

For how the suction component 315 achieves the suction and release of the bioprinting unit C, in some embodiments, in combination with Figs.20-23, the suction component 315 can pre-suck liquid and form liquid film at the suction end of the suction component 315, and the bioprinting unit C is maintained at the suction end of the suction component 315 and is in contact with the liquid film after being sucked by the suction component 315. The suction component in this embodiment is a pipette gun, the suction end is specifically the opening of the pipette gun, the liquid film is formed by gas suction at the opening of the pipette gun, and in other implementation modes, the formation of the liquid film can be implemented by adopting pipette, burettes, syringes and the like as well. The liquid pre-sucked into the suction component 315 is capable of wetting the suction end of the suction component 315, so that liquid film is formed at the suction end of the suction component 315; when the suction component 315 continues to suck the bioprinting unit C, the liquid film can avoid direct contact between the suction end and the bioprinting unit C, thereby preventing the shape and biological activity of the bioprinting unit C from being damaged and destroyed due to rigid contact with the suction end. Moreover, since the liquid film can separate the suction end from the bioprinting unit C, even the bioprinting unit C with a particle diameter smaller than the suction port diameter of the suction end can be held at the suction port under the action of the liquid film and negative pressure, therefore, the liquid film can also be provided to effectively meet the particle size requirements of the non-inhalation micro-particle acquisition method for the bio-printing unit C, and expand the application scope of this non-inhalation micro-particle acquisition method, so that more specifications and more types of bio-printing units C can be manipulated under the premise of minor damage; due to the relatively small size of the bioprinting unit C, the number of cells contained therein is relatively small, and the demand for nutrient supply is relatively low, therefore the use of liquid film to achieve non-inhalation acquisition of the bioprinting unit C with a relatively small particle size is also beneficial to better maintain the biological activity of the bioprinting unit C during the printing process, and helps to finally obtain a biological construct with better biological activity.

In addition, the liquid film pre-formed at the suction end can achieve an attraction effect on the bioprinting unit C that is sucked later, so that the bioprinting unit C is not only subjected to negative pressure, but is simultaneously subjected to the surface tension of the liquid film; on the one hand, this can reduce the negative pressure required by the suction component 315, not only can reduce the technical requirements of suction, improve the probability of success of suction, but also can reduce the damage caused by the suction force to the bioprinting unit C; on the other hand, it can also make the bioprinting unit C be retained more firmly at the suction port of the suction end, thereby enhancing the firmness of the suction process and allowing the bioprinting unit C to be retained firmly at the opening of the pipette during the displacement process, and the bioprinting unit C is not prone to falling off, and the stability and reliability of the displacement process are improved; on the other hand, by virtue of the liquid film, the bioprinting unit C can be kept in a liquid environment all the time from it is sucked out until it is placed in a set printing position, and the liquid environment is an important factor for the bioprinting unit C to maintain the biological activity, therefore, by arranging the liquid film, the bioprinting unit C can still maintain good activity even during the displacement process, and thus the success rate of bioprinting is increased.

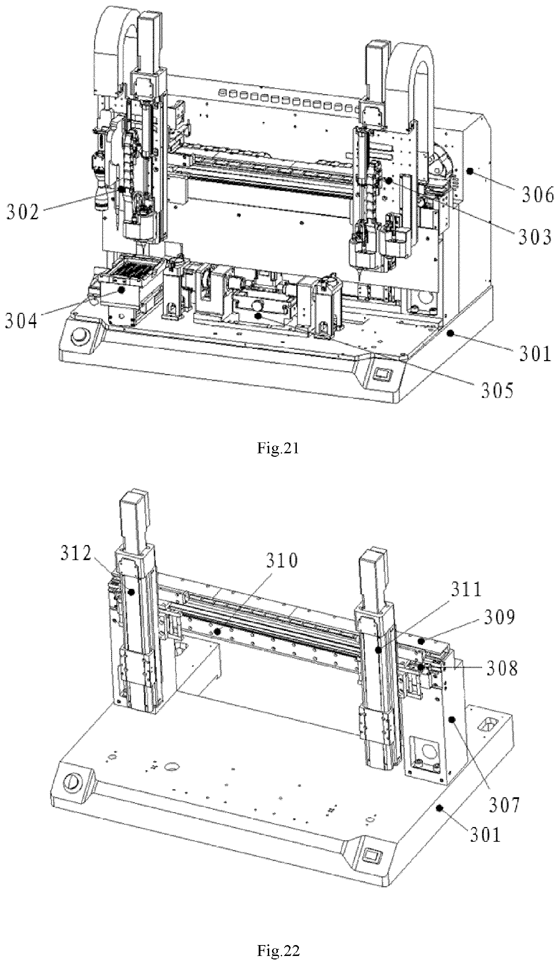

In order to cooperate with the suction component 315 to suck only a single bioprinting unit C at a time to achieve high-precision printing, in some embodiments, as shown in Figs.21, 25 and 26, the biological construct printing device further includes a printing unit kit for storing the bioprinting units C; the printing unit kit 304 is internally provided with a plurality of hole positions 324; and each hole position 324 is used to store a single bioprinting unit C.

In order to achieve precise capture of the bioprinting unit C so as to accurately position the suction component 315, as shown in Figs. 17, 23 and 26, the biological construct printing device may further include a control mechanism and a vision system 314 which is used to capture the position of the bioprinting unit C inside the printing unit kit 304; the vision system 314 can send a feedback signal to the control mechanism after capturing a single bioprinting unit C; the control mechanism manipulates the suction component 315 to move to a corresponding position inside the printing unit kit 304 according to the feedback signal and suck the bioprinting unit C, and then manipulates the suction component 315 to move to the position of the printing unit bonding layer B to release the bioprinting unit C. In some embodiments, the vision system 314 includes a CCD camera capable of easily realizing precise capture of the position of the bioprinting unit C in the printing unit kit 304, and then the position signal is fed back to the control mechanism to realize automatic digital control, which is beneficial to improve the printing efficiency.

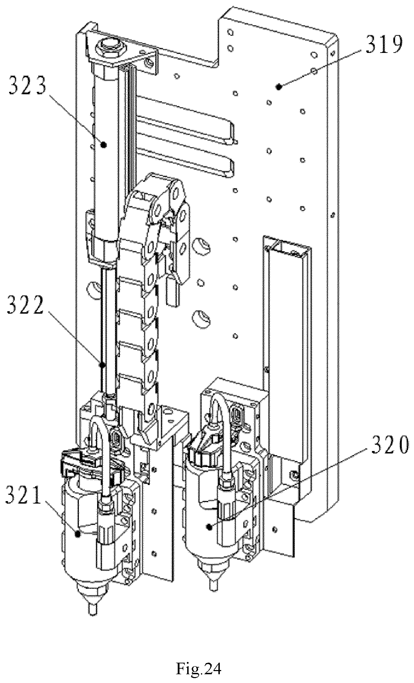

In some embodiments, as shown in Fig.24, the biological construct printing device further includes a third coating component 320 for spraying a molding material or a support material on the surface of the biological construct; by providing the third coating component 320, the surface of the printed and shaped biological construct is sprayed with the molding material or the support material for protecting or supporting the printed and shaped biological construct.

In the above-mentioned embodiment, the biological construct may be a non-lumen-like structure, in which case the support is a planar base body; the biological construct may also be a lumen-like structure, in which case the support 329 is a rotating rod, and the predetermined area is the outer wall of the rotating rod. The biological construct printing device of the present disclosure is particularly suitable for printing a lumen-like structure; the lumen-like structure, for example, is a blood vessel precursor; and the molding material or support material includes a biocompatible material.

As shown in Fig.18 and Fig.19, in the printing process adopting the biological construct printing device of the present disclosure, for the sequential arrangement of the bioprinting units C, a "circumferential arrays first, then linear arrays" arrangement way shown in Fig.18 can be selected, and a "linear arrays first, then circumferential arrays" arrangement way can be selected as well.

The structure of the biological construct printing device and the process of printing the biological construct of the present disclosure will be described below by taking the lumen-like structure as a blood vessel precursor and the support 329 as a rotating rod as an example in combination with the embodiments shown in Figs.21-27.

As shown in Fig.21, the main structure of the biological construct printing device includes a base 301, a first nozzle assembly 302, a second nozzle assembly 303, a printing unit kit 304, a rotary biochemical analyzer 305, and an electric cabinet 306. The electric cabinet 306 is internally provided with a control mechanism. As shown in Fig.6, the base is provided with an upright column 307; a grating scale 308, a guide rail 310, a linear motor 309 a Z-direction movement module 312 and a second Z-direction movement module 311 are provided on the upright column 307. The Z-direction movement module 312 and a second Z-direction movement module 311 are correspondingly used to implement lift control on the first injector 302 and the second injector 303.

As shown in Fig.7, the first nozzle assembly 302 mainly includes a first fixed vertical plate 313, a vision system 314, a suction component 315, a second coating component 316 (preferably a injector), a first driving guide rail 317, and a first driving cylinder 318. The vision system 314, the suction component 315, the second coating component 316, the first driving guide rail 317 and the first driving cylinder 318 are disposed at one side of the upright column 307 through the first fixed vertical plate 313. As shown in Fig.24, the second nozzle assembly 303 mainly includes a second fixed vertical plate 319, a third coating component 320, a first coating component 321, a second driving guide rail 322, and a second driving cylinder 323. The third coating component 320, the first coating component 321, the second driving guide rail 322, and the second driving cylinder 323 are disposed at the other side of the upright column 307 through the second fixed vertical plate 319.

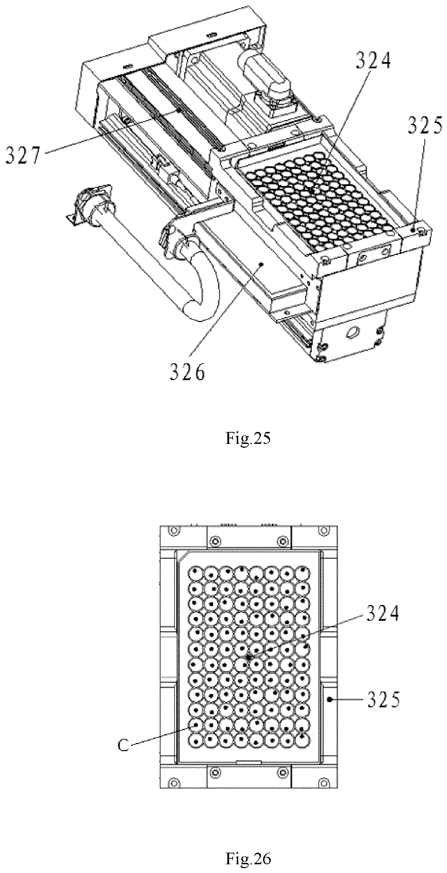

As shown in Fig.25 and Fig.26, the printing unit kit 304 mainly includes an orifice plate bracket 325, an orifice plate, a perspective light source, a driving motor 326 and a third driving guide rail, wherein the orifice plate is located inside the orifice plate bracket 325 and a plurality of hole positions 324 are formed inside the orifice plate. The function of the perspective light source is to provide perspective light so that the vision system 314 can detect the bioprinting unit C more easily under the action of the light source. As shown in Fig.27, the rotary biological analyzer 305 mainly includes a support base 328, a rotating motor 330, a support 329 (a rotating rod), and scraper ropes 331 which are disposed at the two sides of the support base 328. The rotating motor 330 is used to drive the rotating rod to rotate to facilitate adjustment of the printing position. The scraping ropes 331 are used to scrape the excess material liquid sprayed out of the first coating component 321, the second coating component 316 and the third coating component 320, thereby helping to avoid blocking.

During the printing process, firstly, the first nozzle assembly 302 is moved upwards to a safe distance along the Z axis, so that the second coating component 316 will not hit any object on the base 301 when it is lowered. Then, under the action of the first driving cylinder 318, the second coating component 316 moves down to a bottom limitation position along the first driving guide rail 317. Under the action of the linear motor 309, the first nozzle assembly 302 moves in the X direction along the guide rail 310 to the printing range of the support 329, and the printing range corresponds to the predetermined area of the elastic film A sleeved on the support 329. Under the control of the first Z-direction movement module 312, the first nozzle assembly 302 moves down along the Z axis to the printing height, and the second coating component 316 ejects the isolation medium (temperature-sensitive hydrogel) to form an isolation medium layer D on the elastic film A. After spraying is completed, the second coating component 316 moves up to a top limitation position along the first driving guide rail 317 under the action of the first driving cylinder 318, and the first nozzle assembly 302 returns to the position of the left side.

Secondly, the second nozzle assembly 303 moves up to a safe position along the Z axis so that the first coating component 321 will not hit any object below when it is lowered, and then under the action of the second driving cylinder 323, the first coating component 321 moves down to a bottom limitation position along the second driving guide rail 322. Under the action of the linear motor 309, the second nozzle assembly 303 moves in the X direction along the guide rail 310 to the printing range of the support 329, the second nozzle assembly 303 moves downward in the Z direction to the printing height under the control of the second Z-direction movement module 311, and the first coating component 321 ejects the first reagent to form the printing unit bonding layer B as shown in Fig.17. After spraying is completed, the first coating component 321 moves up to top limit position along the second driving guide rail 322 under the action of the second driving cylinder 323, and the second nozzle assembly 303 returns to the position of the right side.

After two layers of materials (namely the printing unit bonding layer B and the isolation medium layer D) are sprayed on the elastic film A, bonding the bioprinting unit C will be performed. Firstly, the vision system 314 is used to position the bioprinting unit C. Since the vision system 314 does not recognize stacked objects, each bioprinting unit C needs to be placed in a separate hole position 324 so as to prevent stacking. This is done when the bioprinting unit C is prepared, thereby ensuring that there is only one bioprinting unit C in each hole position 324. At the moment, the center of a camera lens in the vision system 314 is aligned to the circle range on the hole position 324, and there is only one bioprinting unit C in this range, so the bioprinting unit C can be quickly recognized and accurately positioned. The specific implementation is as follows: after the program is set, the center of the camera lens in the vision system 314 is aligned to the first hole position 324 in the upper left of the orifice plate, the camera lens moves in the Y direction along the third driving guide rail 327 by the driving motor 326, and the first nozzle assembly 302 moves in the X direction. The first nozzle assembly 302 moves in the Z direction to keep a predetermined distance between the top end of the camera lens and a captured object, at the moment, the bioprinting unit C is just within the focal length range of the camera, and there is only one bioprinting unit C in the range, therefore, quick capture and positioning of the bioprinting unit C is realized.

After the center position of the first nozzle assembly 302 is obtained, the control mechanism automatically converts the X and Y coordinate values of the suction component 315. Under the action of the control mechanism, the driving motor 326 moves in the Y direction along the third driving guide rail 321, and the first nozzle assembly 302 moves in the X direction to make sure that the suction component 315 is aligned to the center of the captured bioprinting unit C. Meanwhile, after the first nozzle assembly 302 moves downward along the Z axis to a set distance, as shown in Fig.20, the suction function of the suction component 315 is started to suck the bioprinting unit C so as to prevent the bioprinting unit C from falling off. The first nozzle assembly 302 moves up to a safe position along the Z direction and then moves in the X direction to a position where the bioprinting unit C needs to be placed on the support 329; the first nozzle assembly 302 moves down along the Z axis to a set height in the program, the gas blowing function of the suction component 315 is automatically is started, the bioprinting unit is placed on the elastic film A sleeved on the support 329, and then the placement of the first bioprinting unit is completed. The previous actions are repeated; when the second bioprinting unit C is placed, according to the difference between the bioprinting unit C and the arrangement requirements, the rotating rod acting as the support 329 will automatically move a corresponding distance to achieve precise arrangement on the elastic film A. In a similar way, repeated printing shown in Fig.18 and Fig.19 is realized; until the surface of the rotating rod is full of arranged bioprinting units C, the placement of the bioprinting units C is ended, and the first nozzle assembly 302 returns to the position of the left side.

Finally, the second nozzle assembly 303 is moved, the third coating component 320 is moved to the printing position, the molding material or the supporting material (for example, the biocompatible material at the outermost layer) is sprayed, and the process of printing the biological construct is completed.

"Step n" mentioned herein only represents the step name of step n; if the sequential order of each step is not clearly indicated, the serial number of each step and the description order of each step all do not represent the necessary sequential order of each step, when two steps are described in sequence, there may be no other steps between the two steps or other steps can also be arranged between the steps.

The present disclosure refers to a plurality of embodiments, as long as no contradiction occurs between different embodiments, the technical characteristics of all the embodiments may be combined and/or replaced as needed.

The embodiments combined above make detailed descriptions of the implementation modes of the present disclosure, but the present disclosure is not limited to the described implementation modes. For those skilled in the art, various changes, modifications, equivalent replacements and variations to these implementation modes without departing from the present disclosure still fall within the protection scope of the present disclosure.

## Claims

1. A manufacturing method of a lumen tissue construct, comprising:
step 1: installing an elastic film on a support in a sleeving manner;
step 3: printing a biological construct on the elastic film;
step 5: forming a construct bonding layer on the biological construct; and
step 7: bonding a lumen tissue to the construct bonding layer.

2. The manufacturing method of the lumen tissue construct according to claim 1, wherein the step 3 comprises:
step 31: providing at least one bioprinting unit;
step 33: forming a printing unit bonding layer on a predetermined area of the elastic film; and
step 35: bonding the bioprinting unit to the printing unit bonding layer to form a biological construct or an intermediate structure of the biological construct.

3. The manufacturing method of a lumen tissue construct according to claim 2, wherein the step 33 comprises: forming an isolation medium layer on a predetermined area of the elastic film, and then forming the printing unit bonding layer on the elastic film through the isolation medium layer.

4. The manufacturing method of the lumen tissue construct according to claim 2, wherein the step 3 further comprises:
step 37: forming a printing unit bonding layer on the intermediate structure obtained in the previous step to obtain an intermediate structure comprising a printing unit bonding layer; and
step 39: bonding the bioprinting unit to the intermediate structure comprising the printing unit bonding layer.

5. The manufacturing method of the lumen tissue construct according to claim 4, wherein the step 3 comprises: repeating step 37 and step 39 one or more times.

6. The manufacturing method of the lumen tissue construct according to claim 1, wherein step 5 comprises: forming a support layer on the surface of the biological construct, and then forming a construct bonding layer on the biological construct through the support layer.

7. The manufacturing method of the lumen tissue construct according to claim 1, wherein the step 5 comprises: printing a bonding agent on the biological construct to form the construct bonding layer.

8. The manufacturing method of the lumen tissue construct according to claim 1, wherein the biological construct is a lumen structure, and the support is a rotating rod.

9. The manufacturing method of the lumen tissue construct according to claim 1, wherein the step 7 comprises:
installing the lumen tissue outside a hollow rod in a sleeving way;
installing the hollow rod outside the biological construct provided with the construct bonding layer in a sleeving way;
retaining the lumen tissue outside the biological construct while pulling out the hollow rod; and
bonding the lumen tissue to the construct bonding layer.

10. The manufacturing method of the lumen tissue construct according to claim 9, wherein bonding the lumen tissue to the construct bonding layer comprises: expanding the elastic film to increase the outer diameter of the biological construct and the outer diameter of the construct bonding layer, so that the construct bonding layer is attached to the inner wall of the lumen tissue.

11. The manufacturing method of the lumen tissue construct according to claim 10, wherein conveying gas from the inside of the support to the surface of the support to expand the elastic film.

12. The manufacturing method of the lumen tissue construct according to claim 1, further comprising heating the support.

13. The manufacturing method of the lumen tissue construct according to claim 1, further comprising detecting the flatness of the inner wall of the biological construct.
